# EUROPEAN PATENT SPECIFICATION

(11) **EP 3 066 210 B1**
(45) Date of publication and mention of the grant of the patent: **05.12.2018**
(21) Application number: 14792764.4
(22) Date of filing: 16.10.2014
(51) Int. Cl.: C12Q 1/689

(54) **DETECTION OF STREPTOCOCCUS PNEUMONIAE**
DETEKTION VON STREPTOCOCCUS PNEUMONIAE
DÉTECTION DE STREPTOCOCCUS PNEUMONIAE

(30) Priority: 17.10.2013 EP 13382411
(43) Date of publication of application: 14.09.2016
(73) Proprietor: Genomica S.A.U., 28033 Madrid (ES)
(72) Inventor: MORAGA QUINTANILLA, Ana Isabel, 28823 Coslada (Madrid) (ES); VILLAHERMOSA JAEN, Maria Luisa, 28823 Coslada (Madrid) (ES)
(74) Representative: Bailey, Jennifer Ann
(86) International application number: PCT/EP2014/072227
(87) International publication number: WO 2015/055768

(56) References cited:
- WO-A2-2004/041841
- WO-A2-2005/059156
- US-A- 5 738 987
- M. H. LEUNG ET AL: "Sequetyping: Serotyping Streptococcus pneumoniae by a Single PCR Sequencing Strategy", JOURNAL OF CLINICAL MICROBIOLOGY, vol. 50, no. 7, 2 May 2012 (2012-05-02), pages 2419-2427, XP055110142, ISSN: 0095-1137, DOI: 10.1128/JCM.06384-11
- IKRYANNIKOVA L N ET AL: "Misidentification of alpha-hemolytic streptococci by routine tests in clinical practice", INFECTION, GENETICS AND EVOLUTION, ELSEVIER, AMSTERDAM, NL, vol. 11, no. 7, 8 July 2011 (2011-07-08), pages 1709-1715, XP028307637, ISSN: 1567-1348, DOI: 10.1016/J.MEEGID.2011.07.010 [retrieved on 2011-07-17]
- M. J. FERRANDIZ ET AL: "New Mutations and Horizontal Transfer of rpoB among Rifampin-Resistant Streptococcus pneumoniae from Four Spanish Hospitals", ANTIMICROBIAL AGENTS AND CHEMOTHERAPY, vol. 49, no. 6, 25 May 2005 (2005-05-25), pages 2237-2245, XP55163241, ISSN: 0066-4804, DOI: 10.1128/AAC.49.6.2237-2245.2005
- "AY695461", EBI , 1 June 2005 (2005-06-01), Retrieved from the Internet: URL:http://www.ebi.ac.uk/cgi-bin/sva/sva.p l?query=AY695461&snapshot=&session=%2Ftmp% 2FSESSION8522-1486627817-1&comp_id=2870365 745&comp_id=310803309&compare=Compare+Sele cted&format=EMBL [retrieved on 2017-02-09]

## Description

### Background of the invention.

*Streptococcus pneumoniae* is a causative agent of sepsis, and it would be convenient to be able to specifically detect its presence in blood. *Streptococcus pneumoniae* is also a major cause of community-acquired pneumonia. It is of great importance to be able to distinguish *Streptococcus pneumoniae* from the other members of the *Streptococci* group, in order to determine whether any *Streptococcus* present in a sample corresponds actually to pathogenic *Streptococcus pneumoniae* species, or to other innocuous *Streptococcus* species constitutively present in the host, and which do not cause disease.

Many attempts have been carried out in order to detect *Streptococcus pneumoniae* and distinguish it from the other members of the *Streptococci* group (Wessels et al., J. Clin. Microbiol. April 2012 vol. 50 no. 4 1171-1177). These include culture testing and conventional biochemical and phenotypic tests, such as Gram stain morphology, optochin susceptibility, colony morphology, and alpha-hemolysis on sheep blood agar. Further techniques that are used include: MALDI-TOF (MS matrix-assisted laser desorption ionization-time of flight mass spectrometry), sequence analysis of several genes, and molecular assays including Real-Time PCR assay. Most of these techniques, however, do not allow discriminate *Streptococcus pneumoniae* out of other members of the *Streptococci* group, due to the high sequence similarity between the different species. Other techniques include molecular methods based on amplification of the region of the gene rpoB for detection of bacteria of the genus *Streptococcus.* Two particular examples of this approach are provided in EP 1558637 and EP 1694861.
EP 1558637 makes use of a 724 bp fragment of the rpoB gene (displayed as SEQ ID N° 12 in WO 2004/041841), for detection of bacteria of the *Streptococcus* genus that may be present in a sample. Such detection may take place through amplification of the indicated fragment. No particular probe is, however, provided for the specific detection of different species of the *Streptococcus* genus.
In EP1694861 a fragment of the rpoB gene is amplified with a pair of primers that is common to all the species of the *Streptococcus* genus. An amplification product is thereby obtained, irrespective of the particular species that may be present in the sample, as in EP 1558637 above. In the present case probes are provided, which are intended for the specific detection of *Streptococcus pneumoniae* and *Streptococcus pyogenes* (Table 3 of corresponding WO 2005/059156). The minimal sequence differences between some of the probes, however, may prove difficult that such probes may actually be useful for a specific detection of the two different *Streptococcus* species they target (e.g.: probes of SEQ ID N° 4 and SEQ ID N° 5, corresponding to *Streptococcus pyogenes* and *Streptococcus pneumoniae,* respectively, just differ in two nucleotides). No additional probes corresponding to other species of the *Streptococcus* genus are provided.

In view of the information above, there is a need to provide effective methods based on probe hybridization for the specific detection of *Streptococcus pneumoniae vs* other species of the *Streptococcus* genus. In other words, it is highly desirable to provide a method that allows differential detection of *Streptococcus pneumoniae* present in a sample, that is specific detection of *Streptococcus pneumoniae* distinguished from other species of the *Streptococcus* genus.

### Summary of the invention.

The problem to be solved by the present invention is the provision of a method for the detection of *Streptococcus pneumoniae* present in a sample, which allows to specifically distinguish *Streptococcus pneumoniae* from other species of the *Streptococcus* genus.

The solution is based on the simultaneous use of probes comprising or consisting of SEQ ID N° 1 and SEQ ID N° 2 (see Table 1 below) to detect the presence of *Streptococcus pneumoniae* in a sample. In one embodiment, the probe consists of SEQ ID N° 1 or SEQ ID N° 2.

As indicated above, there is a high sequence similarity between the different species of the *Streptococcus* genus; this circumstance constitutes a heavy burden when trying to detect *Streptococcus pneumoniae* and specifically distinguish it from other *Streptococcus* species, through probe hybridization. The probes of SEQ ID N° 1 and SEQ ID N° 2 and thus the various aspects of the invention allow to efficiently detect *Streptococcus pneumoniae* and distinguish *Streptococcus pneumoniae* from, at least, the following species of the *Streptococcus* genus: *Streptococcus (S.) constellatus, S. anginosus, S. mitis, S. gallolyticus, S. dysgalactiae, S. pyogenes, S*. *suis, S*. *intermedius, S. sanguinis, S. oralis and S*. *parasanguinis.*

According to the various aspects of the invention, simultaneous detection through hybridization with the two probes as defined in SEQ ID N° 1 and SEQ ID N° 2 is carried out for the specific detection of *Streptococcus pneumoniae.* Only when DNA present in a sample corresponds to *Streptococcus pneumoniae,* binding to both of these probes takes place. In cases where none or only one of the probes of SEQ ID N° 1 and SEQ ID N° 2 binds DNA present in a sample, this DNA does not correspond to *Streptococcus pneumoniae,* but to other species of the *Streptococcus* genus instead. The only exception is the case of a sample containing DNA of *Streptococcus mitis,* in which case sample DNA binds to probe of SEQ ID N° 1, binding of DNA to probe of SEQ ID N° 2 also taking place to a certain level, but to a much lower extent than to probe of SEQ ID N° 1 (see Example 2 below). A "much lower extent" it is understood to mean a binding level of 20% or lower than the one achieved with SEQ ID N° 1.

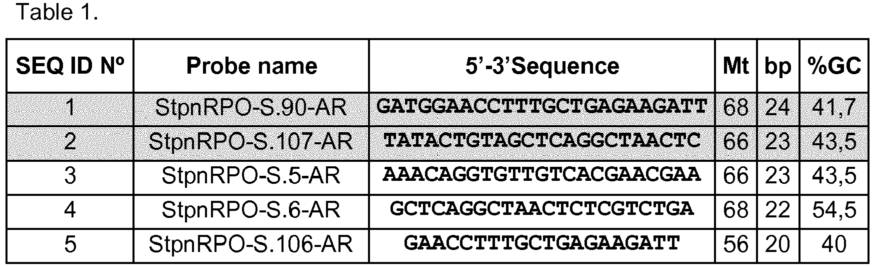

Probes as defined by SEQ ID N° 3, SEQ ID N° 4 and SEQ ID N° 5 of Table 1 also bind nucleic acids of *Streptococcus pneumoniae.* However, they have proven to be nonspecific, as they also arbitrarily bind nucleic acids of other species of the *Streptococcus* genus, without following any kind of predictable pattern.

The inventors are not aware that there is any pointer in the state of the art nor in the probes sequences to explain why simultaneous hybridization with probes of SEQ ID N° 1 and SEQ ID N° 2 would lead to detection of *Streptococcus pneumoniae,* in a way that would allow to distinguish it in a specific way from the other species of the *Streptococcus* genus. There is no explanation to be found either in the Melting Temperature, number of nucleotides, %GC (Table 1), or in the number of nucleotides by which these probes differ from the *Streptococcus pneumoniae* wild-type sequence (Table 2).

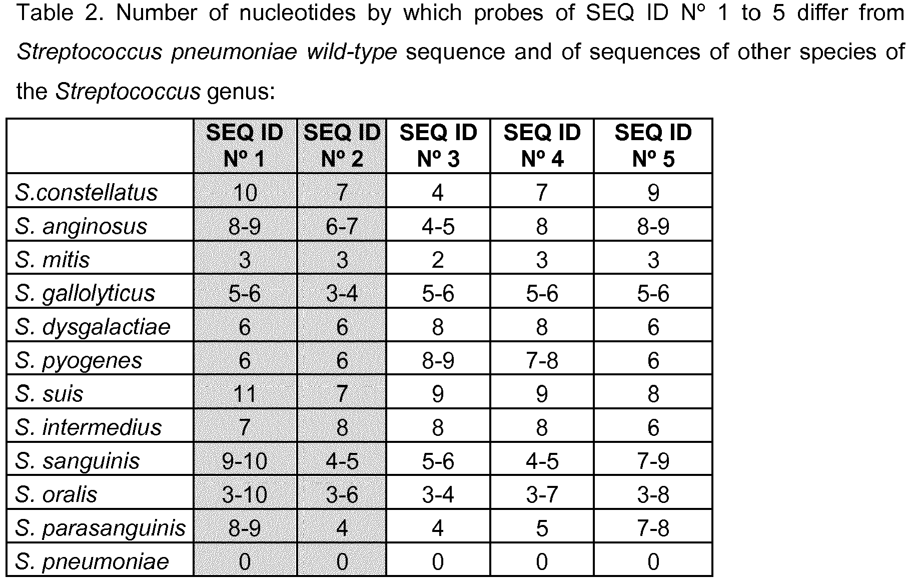

Different variants of the sequence of certain species have sometimes been detected. This is the reason why sometimes the number of nucleotides by which a certain probe differs from the sequence of a certain species of *Streptococcus* ranges between two values.

The inventors have thus surprisingly found that simultaneous incubation with probes as defined by SEQ ID N° 1 and SEQ ID N° 2 allows to detect and to specifically distinguish *Streptococcus pneumoniae* from other species of the *Streptococcus* genus. Thus, simultaneous use of probes of SEQ ID N° 1 and SEQ ID N° 2 provides a solution to the problem of specific detection of *Streptococcus pneumoniae* in a sample. This is of great importance to distinguish *Streptococcus pneumoniae* from other microorganisms; in particular, from innocuous *Streptococcus* species constitutively present in the host and which do not cause disease, in particular, in the respiratory track. This is also important for the detection of the presence of *Streptococcus pneumoniae* in blood as a causative agent of sepsis.

One of the aspects of the present invention thus corresponds to a method for the detection of *Streptococcus pneumoniae* in a test sample, said method being defined by claim 1.

Embodiments of the various aspects of the invention are defined by the dependent claims.

A second aspect of the present invention corresponds to a kit for the detection of *Streptococcus pneumoniae* in a test sample, wherein said kit comprises an array vessel or set of array vessels, each comprising a microarray wherein both probes of SEQ ID N° 1 and SEQ ID N° 2 are provided, and an amplification mixture with PCR amplification primers which yield amplification products that include the region that hybridizes with probes of SEQ ID N° 1 and SEQ ID N° 2.

Third and fourth aspects of the present invention respectively correspond to a set of probes comprising probes SEQ ID N° 1 and SEQ ID N° 2, and to a microarray comprising this set of probes. Also described is an isolated nucleic acid sequence consisting of SEQ ID N° 1 and a microarray comprising SEQ ID N° 1. The isolated nucleic acid sequence may be labelled with a detectable moiety. Further described is an isolated nucleic acid sequence consisting of SEQ ID N° 2 and a microarray comprising SEQ ID N° 2. The isolated nucleic acid sequence may be labelled with a detectable moiety. Also disclosed is a composition comprising SEQ ID N° 1 and/or SEQ ID N° 2.

Further aspects of the present invention correspond to use of the kit, microarray, isolated nucleic acid sequence or set of probes of the invention for the *in vitro* detection of *Streptococcus pneumoniae,* and for diagnosis of an infection by *Streptococcus pneumoniae* in a patient sample. In particular, infection is a septic or respiratory infection.

A method for diagnosing an infection by *Streptococcus pneumoniae* in a patient comprises contacting a sample from said patient with probes as defined by SEQ ID N° 1 and SEQ ID N° 2.

### Brief description of the drawings.

Figure 1.

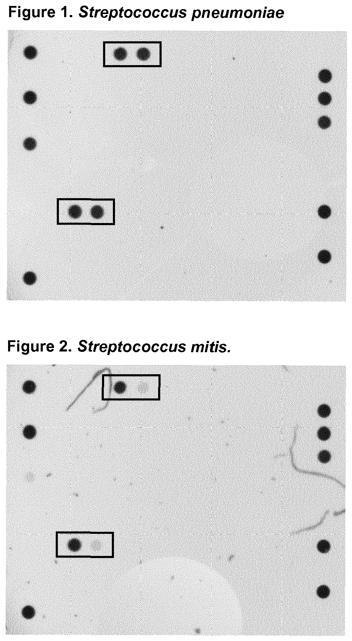

   Figure 1 shows visualization of hybridization of the amplification products of Example 1 of the present invention, previously denatured, with a microarray which comprises probes of SEQ ID N° 1 and SEQ ID N° 2 of the present invention.
   The "spots" surrounded by rectangles correspond to positions of the probes of SEQ ID N° 1 and SEQ ID N° 2. Of the two "spots" surrounded by each rectangle, the first one corresponds to SEQ ID N° 1, and the second one to SEQ ID N° 2. The "spots" outside the rectangles that display a signal, correspond to the Position Markers.
   The sample of Example 1, which contains *Streptococcus pneumoniae,* displays a positive signal and of equal intensity, in both the positions corresponding to probes of SEQ ID N° 1 and SEQ ID N° 2. This is due to the specific binding to both these probes of the amplified and denatured DNA of the sample.
Figure 2.
   Figure 2 displays visualization of hybridization of the amplification products of Example 2 of the present invention, previously denatured, with a microarray which comprises probes of SEQ ID N° 1 and SEQ ID N° 2 of the present invention.
   The "spots" surrounded by rectangles correspond to positions of the probes of SEQ ID N° 1 and SEQ ID N° 2. Of the two "spots" surrounded by each rectangle, the first one corresponds to SEQ ID N° 1, and the second one to SEQ ID N° 2. The "spots" outside the rectangles that display a signal, correspond to the Position Markers.
   The sample of Example 2, which contains *Streptococcus mitis,* displays a positive signal in the position corresponding to probe of SEQ ID N° 1, an a signal of much lower intensity in that of probe of SEQ ID N° 2. The signal that appears in position of SEQ ID N° 2 has an intensity of less than 20% of the one that appears in position of SEQ ID N° 1.
Figure 3.

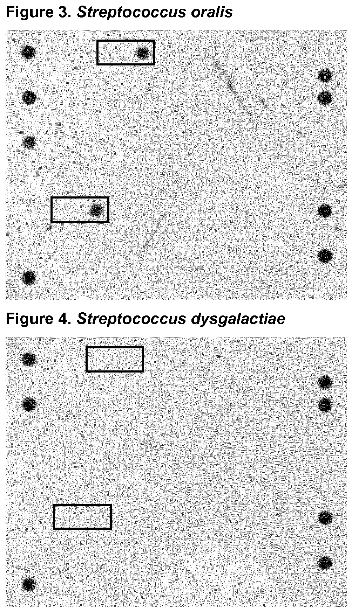

   Figure 3 shows visualization of hybridization of the amplification products of Example 3 of the present invention, previously denatured, with a microarray which comprises probes of SEQ ID N° 1 and SEQ ID N° 2 of the present invention.
   The "spots" surrounded by rectangles correspond to positions of the probes of SEQ ID N° 1 and SEQ ID N° 2. Of the two "spots" surrounded by each rectangle, the first one corresponds to SEQ ID N° 1, and the second one to SEQ ID N° 2. The "spots" outside the rectangles that display a signal, correspond to the Position Markers.
   The sample of Example 3, which contains *Streptococcus oralis,* displays a positive signal only in the position corresponding to probe of SEQ ID N° 2, and does not display a signal in the position of the probe of SEQ ID N° 1.
Figure 4.
   Figure 4 shows visualization of hybridization of the amplification products of Example 4 of the present invention, previously denatured, with a microarray which comprises probes of SEQ ID N° 1 and SEQ ID N° 2 of the present invention.

The "spots" surrounded by rectangles correspond to positions of the probes of SEQ ID N° 1 and SEQ ID N° 2. Of the two "spots" surrounded by each rectangle, the first one corresponds to SEQ ID N° 1, and the second one to SEQ ID N° 2. The "spots" outside the rectangles that display a signal, correspond to the Position Markers.

The sample of Example 4, which contains *Streptococcus dysgalactiae,* does not display any kind of signal in the positions of probes of SEQ ID N° 1 nor SEQ ID N° 2.

### Detailed description of the invention.

In the following passages, different aspects of the present invention are defined in more detail. Each aspect so defined may be combined with any other aspect or aspects unless clearly indicated to the contrary. In particular, any feature indicated as being preferred or especially advantageous may be combined with any other preferred or advantageous feature or features.

A first aspect of the present invention corresponds to a method for the detection of *Streptococcus pneumoniae* in a test sample, the method being defined by claim 1.

A second aspect of the present invention relates to a kit for the detection of *Streptococcus pneumoniae* in a test sample, wherein said kit comprises an array vessel or set of array vessels, each comprising a microarray wherein both probes of SEQ ID N° 1 and SEQ ID N° 2 are provided, and an amplification mixture with PCR amplification primers which yield amplification products that include the region that hybridizes with probes of SEQ ID N° 1 and SEQ ID N° 2.

Third and fourth aspects of the present invention respectively correspond to a set of probes comprising probes of SEQ ID N° 1 and SEQ ID N° 2, and to a microarray comprising this set of probes. Another aspect relates to an isolated nucleic acid sequence consisting of SEQ ID N° 1. Another aspect relates to an isolated nucleic acid sequence consisting of SEQ ID N° 2.

Further aspects of the present invention correspond to use of method, kit, microarray, isolated nucleic acid sequence or set of probes of the invention for the detection of *Streptococcus pneumoniae,* and for diagnosis of an infection by *Streptococcus pneumoniae* in a patient. In particular, infection is a septic or respiratory infection.

A method for diagnosing an infection by *Streptococcus pneumoniae* in a patient comprises contacting a sample from said patient with probes as defined by SEQ ID N° 1 and SEQ ID N° 2. The method may comprise an amplification step as further explained below. If both probes hybridise to DNA present in the sample, then the patient is diagnosed as having an infection caused by *Streptococcus pneumonia.* The method is preferably carried out *in vitro* or *ex vivo.*

Also disclosed is an assay for the detection of *Streptococcus pneumoniae* in a test sample, the method comprising contacting the sample with probes of SEQ ID N° 1 and SEQ ID N° 2.

### Definitions:

The terms "probes of/as defined by SEQ ID N° 1 and SEQ ID N° 2" as used herein refer to both the nucleotide sequences depicted as SEQ ID N° 1 and SEQ ID N° 2, but also obvious equivalents to them, such as their complement sequences or sequences that have equivalent binding ability to that of SEQ ID N° 1 and SEQ ID N° 2 or that of their complement sequences (e.g. sequences that differ from sequences of SEQ ID N° 1 and 2 or from their complement sequences by 1 mutation thereof).

"Primer", "Amplification Primer" or "PCR Amplification Primer" as used herein mean "primer of a nucleic acid amplification reaction". A "much lower extent of binding" as used hereint is understood to mean a binding level of 20% or lower than the one to which reference is made to. Binding of the DNA to be tested and any probe takes place through hybridization.

In preferred embodiments, the probes are provided in a microarray, for example immobilized on a solid support. A microarray is a collection of microscopic oligonucleotide spots. A DNA microarray (also commonly known as gene chip, DNA chip, or biochip) is a collection of microscopic DNA spots attached to a solid surface. Probes are synthesized and then attached via surface engineering to a solid surface by a covalent bond to a chemical matrix (via epoxy-silane, amino-silane, lysine, polyacrylamide or others). Solid surfaces are known in the art and include microscopic beads as well as solid supports.

Thus, in particular, the probes of the present invention may be immobilized on a solid support. This solid support can be the bottom of an array vessel or a different solid support attached to the bottom of an array vessel. This means that the surface of the microarray may be the flat bottom of the array vessel. Alternatively, the surface of the microarray may be a solid support attached to the bottom of the array vessel. The probes of the present invention may be included in an individual array vessel or in a set of array vessels.

Described herein and according to the invention, probes are contained in a microarray, which may be placed on a slide or contained in a reaction vessel, which is then called an array vessel. Array vessels may have different formats of presentation, including individual array vessels, such as wells or tubes, or sets of array vessels arranged in strips of wells or tubes, or flat plates. Usually, plates consist of sets of strips of array vessels. Thus, the microarray of the present invention may be contained in an individual array vessel. Alternatively, two or more microarrays may be contained in a strip of vessels. In a preferred embodiment, the strip of vessels is constituted by 8 vessels. Further, three or more array vessels may be arranged in a set of strip of vessels. In another preferred embodiment, the set of strip of vessels is a microtiter plate. In yet another preferred embodiment, the microtiter plate is constituted by 96 array vessels.
The probes of the present invention can be obtained by different methods, such as chemical synthesis (e. g. by the conventional phosphotriester method); or genetic engineering techniques, for example by molecular cloning of recombinant plasmids in which corresponding nucleotide sequences have been inserted and can be latter obtained by digestion with nucleases.

According to the methods and kit of the present invention, the test sample may be amplified before being contacted with the probes of SEQ ID N° 1 and SEQ ID N° 2. Should this be the case, any components known by the skilled person to be necessary for nucleic acid amplification and, in particular, for PCR amplification, may also be present within the amplification mixture. Thus, PCR Amplification Primers, appropriate nucleotide triphosphates, such as dATP, dCTP, dGTP, dTTP, and a suitable enzyme for polymerisation may also be present within the mixture of amplification reagents. Any convenient enzyme for polymerisation may be used. In particular, HotStarTaq DNA Polymerase of QIAGEN Multiplex PCR Kit, performs particularly well. This DNA Polymerase is a modified form of Taq DNA polymerase which has no polymerase activity at ambient temperatures, and is activated by a 15-minute incubation at 95°C. Any other enzyme with these characteristics known in the state of the art, may also preferably be used.
Standard thermal cycling conditions may be used for carrying out amplification according to the present invention. Some specially preferred conditions are:

| **NUMBER OF CYCLES** | **TEMPERATURE** | **TIME** |
|---|---|---|
| 1 cycle | 95°C | 15' |
| 40-45 cycles | 95°C | 15-30" |
| | 55-59°C | 15-60" |
| | 68-72°C | 45-60" |
| 1 cycle | 68-72°C | 10' |
| 1 cycle | 4°C | Forever |

Some specially preferred PCR Amplification Primers are nucleic acid sequences which comprise any of the sequences of the group formed by SEQ ID N° 6 and SEQ ID N° 7 displayed in Table 3 below. Most preferably, PCR Amplification Primers consist of nucleic acid sequences SEQ ID N° 6 and SEQ ID N° 7.

**Table 3:**

| **SEQ ID N°** | **Primer name** | **5'-3'Sequence** |
|---|---|---|
| 6 | Stpn5 | **TCTGAGTTGTCTMACAARCG** |
| 7 | Stpn6r | **GAATACATGCYGTCGCAACRGC** |

| | | |
|---|---|---|
| M=A/C; R=A/G; Y=C/T | | |

It is not essential that amplification primers are those of SEQ ID N° 6 and SEQ ID N° 7. Other pairs of amplification primers that yield amplification products that include the region that hybridizes with probes of SEQ ID N° 1 and SEQ ID N° 2, may alternatively be used.
The present invention is compatible with the presence of amplification primers for other microorganisms within the same reaction mixture. Further, it is also compatible with inclusion of pairs of primers for amplification of, at least, one Internal Control, and one Extraction Control.
After the amplification process, preferably, the amplification product obtained is transformed into single-stranded DNA prior to hybridization with the corresponding probe. Most preferably single-stranded DNA is obtained through denaturation of the amplification product, most preferably, through heat-denaturation.

In cases where the sample to be tested is not amplified prior to incubation with probes, the nucleic acid present in the sample may be labelled by any DNA labelling method known in the art.
Also, a label may be introduced in the DNA amplification product during amplification to allow further detection; in particular, a label that provides a signal that may be detected by colorimetric methods, by fluorescent methods, or by any labelling method known in the art. The label can be radioactive, chemiluminescent, luminescent and fluorescent agents. In a preferred aspect, the label that is used is biotin. However, any other kind of label known in the art may be used (eg. digoxigenin). In a preferred aspect, at least one of the primers used is labelled at the 5' end with biotin. Furthermore, labelling of amplified DNA may alternatively be achieved by adding modified nucleotides bearing a label (e.g. biotinylated or digoxigenin dUTP derivatives) to the PCR mixture. In certain embodiments, radioactive labels may be used, as well as fluorophores.
Alternative methods known to the skilled person, that may enable detection of the interaction between any amplification product and its corresponding probe, including methods that imply labelling of the probe, may be used.
The denatured amplification products can be incubated with probes immobilized in a microarray, either by themselves, or mixed with an hybridization solution comprising salts and detergents known by the person skilled in the art to favour interactions between DNA molecules.
Due to the labelling of the sample DNA, wherever sample molecules interact with probe molecules on the surface of the microarray, a reporter reagent binds the label and produces visible signals which may be detected by a detection device. The interacting probe and sample molecules are identified by the location of the signal on the surface of the microarray. In the particular case where sample amplification products are labelled with biotin, the reporter agent can be horseradish peroxidase covalently joined to streptavidin. The latter binds specifically to biotin, and the peroxidase triggers the precipitation of substrates like tetramethylbenzidine (TMB) or o-Dianisidine.
Any other detection method known in the state of the art, such as fluorescence, may be used for detection of the interaction between amplification products and corresponding probes.

Any other reaction that results in a precipitate on array elements, and that can be used to detect the interaction between target and probe molecules, may equally be used.

In a preferred embodiment, visualization of the interactions between target molecules and their corresponding specific or control probes, consists of the following steps:
- First, the image of the array is captured using an optical device;
- Then, the image is analysed;
- Finally, a report containing an interpretation of the result is provided.
Preferably, the image is analysed by means of appropriate software. Any device suitable for this processing can be used.

In a preferred embodiment, types of test samples that can be processed within the present invention are nasopharyngeal exudates, nasopharyngeal washes, sputum, bronchi-alveolar aspirates, and bronchi-alveolar washes. These would be the most suitable samples for diagnosis of respiratory infections. Further samples to be processed according to the present method and kit are blood and blood culture samples, which are most suitable in the case of patients with symptoms of sepsis. Any other body fluid, or tissue obtained from an individual may also be subjected to the method and kit of the present invention. The individual is human.
The test sample may equally be genetic material extracted from the original sample. Extraction of genetic material can be carried out both by automatic as well as by manual extraction techniques of the state of the art.
In a most preferred embodiment, the automatic extraction system is the NucliSENS easyMAG of BioMerieux (EP1694813), which uses magnetic particles in combination with BioMerieux's BOOM® technology for universal isolation of total nucleic acid from a wide range of sample volumes and types. Other techniques of automatic extraction can equally be used.

The skilled person will also be aware of techniques and methods for manual processing of samples to extract nucleic acids; and any such suitable method may be used.
In a particular example, nucleic acids are extracted from the sample to be tested. Preferably, the initial volume is in the range of from 200 µl to 1 ml. After the extraction step, a precipitate is resuspended in a volume of from 25 µl to 80 µl of RNase-free water. In a particular embodiment, 5 µl of the 25 to 80 µl comprising the genetic material extracted from the test sample, are added to a vessel containing the amplification reactants, in a final volume of 50 µl.

"and/or" where used herein is to be taken as specific disclosure of each of the multiple specified features or components with or without the other at each combination unless otherwise dictated. For example "A, B and/or C" is to be taken as specific disclosure of each of (i) A, (ii) B, (iii) C, (iv) A and B, (v) B and C or (vi) A and B and C, just as if each is set out individually herein.

As used herein and in the appended claims, the singular forms "a", "an", and "the" include the plural reference unless the context clearly dictates otherwise.

The examples provided below merely illustrate the present invention and in no way limit the scope of the accompanying claims.

### Examples.

### Example 1:

A sample of blood culture positive for *Streptococcus pneumoniae* was obtained, and DNA was extracted from it by means of NucliSENS easyMAG kit of BioMerieux. The initial volume of blood culture that was used was of 1 ml, while the final volume after elution was of 80 µl.
Next, 5 µl of the eluted volume were taken and subjected to amplification with the following mixture of reagents:

| **Amplification Reagents** | **Final Concentration PCR tube (µM)** |
|---|---|
| Buffer Q 5x | 1X |
| 2X QIAGEN Multiplex PCR Master Mix | 1X |
| Primer SEQ ID N° 6 | 0.8-2 |
| Primer SEQ ID N° 7, labelled with Biotin | 0.8-2 |
| H₂O | Up to a final volumen of 45 µl |

| **NUMBER OF CYCLES** | **TEMPERATURE** | **TIME** |
|---|---|---|
| 1 cycle | 95°C | 15' |
| 40 cycles | 95°C | 15" |
| | 55°C | 15" |
| | 68°C | 45" |
| 1 cycle | 68°C | 10' |
| 1 cycle | 4°C | Forever |

The amplification products were denatured through incubation at 95°C for 10 minutes. After that, 5 µl of denatured PCR product were incubated, in the presence of a standard hybridization solution, with a microarray which comprises probes of SEQ ID N° 1 and SEQ ID N° 2 immobilized in known positions.
After a series of standard washing and blocking steps, an image was taken with the visualization of the final result that is shown in Figure 1.

### Example 2.

A sample of blood culture positive for *Streptococcus mitis* was obtained, and subjected to the same procedure described within Example 1 for *Streptococcus pneumoniae.* Visualization of the final result is displayed in Figure 2.

### Example 3.

A sample of blood culture positive for *Streptococcus oralis* was obtained, and subjected to the same procedure described within Example 1 for *Streptococcus pneumoniae.* Visualization of the final result is displayed in Figure 3.

### Example 4:

A sample of blood culture positive for *Streptococcus dysgalactiae* was obtained, and subjected to the same procedure described within Example 1 for *Streptococcus.*

## Claims

1. A method for the detection of *Streptococcus pneumoniae* in a test sample, the method comprising:
- subjecting the sample to PCR amplification yielding amplification products that include a region that hybridizes with probes of SEQ ID N° 1 and SEQ ID N° 2, wherein a detectable label is introduced into the amplification products;
- transforming any PCR amplification products into single-stranded DNA;
- contacting any single-stranded DNA with both probes of SEQ ID N° 1 and SEQ ID N° 2, said probes being provided in a microarray; and
- detecting a signal provided by the detectable label of any single-stranded DNA which has hybridized with the probes, wherein:
- positive signals of equal intensity in both the positions of the microarray corresponding to the probes of SEQ ID No. 1 and SEQ ID No. 2 is indicative of the presence of *Streptococcus pneumoniae* in the sample; - a positive signal in the position corresponding to the probe of SEQ ID No. 1, and a signal in the position corresponding to the probe of SEQ ID No. 2 having an intensity of less than 20% of that corresponding to the probe of SEQ ID No. 1, is indicative of the presence of *Streptococcus mitis* in the sample; and
- a positive signal in the position corresponding to only one probe, or no signal in either of the positions corresponding to the probes of SEQ ID No. 1 and 2, is indicative of the presence of another species of the *Streptococcus genus.*

2. The method of claim 1 wherein two nucleic acid sequences respectively comprising SEQ ID N° 6 and SEQ ID N° 7 are used as PCR amplification primers.

3. The method of claim 1 or claim 2 wherein the probes of SEQ ID N° 1 and SEQ ID N° 2 are included in an individual array vessel, or in a set of array vessels.

4. A kit for the detection of *Streptococcus pneumoniae* in a test sample, wherein said kit comprises an array vessel or set of array vessels, each comprising a microarray wherein both probes of SEQ ID N° 1 and SEQ ID N° 2 are provided, and
an amplification mixture, comprising PCR amplification primers and a detectable label, for producing labelled amplification products that include the region that hybridizes with probes of SEQ ID N° 1 and SEQ ID N° 2.

5. The kit of claim 4, wherein said kit further comprises reagents for use in visualising hybridisation of nucleic acids to the probes of the microarray.

6. The kit of claim 4 or claim 5 wherein the amplification mixture comprises two nucleic acid sequences, respectively comprising SEQ ID N° 6 and SEQ ID N° 7, as PCR amplification primers.

7. A set of probes comprising probes of SEQ ID N° 1 and SEQ ID N° 2.

8. A microarray comprising the set of probes as defined in claim 7.

9. Use of the kit of claims 4 to 6, the set of probes of claim 7, or the microarray of claim 8 for *in vitro* detection of *Streptococcus pneumoniae.*

10. Use of the kit of claims 4 to 6, the set of probes of claim 7, or the microarray of claim 8 for diagnosis of an infection by *Streptococcus pneumoniae* in a sample from a patient.

11. Use of claim 10 wherein the infection is a septic or respiratory infection.

## Patentansprüche

1. Verfahren zur Detektion von Streptococcus pneumoniae in einer Testprobe, wobei das Verfahren Folgendes beinhaltet:
- Unterwerfen der Probe unter eine PCR-Amplifikation, welche Amplifikationsprodukte ergibt, welche Folgendes beinhalten
eine Region, welche mit Sonden aus SEQ ID Nr. 1 und SEQ ID Nr. 2 hybridisiert, wobei ein detektierbarer Label in die Amplifikationsprodukte eingeführt wird;
- Verwandeln von beliebigen PCR-Amplifikationsprodukten in einsträngige DNS;
- Inkontaktbringen von beliebiger einsträngiger DNS mit beiden Sonden aus SEQ ID Nr. 1 und SEQ ID Nr. 2, wobei die Sonden in einem Mikroarray bereitgestellt werden; und
- Detektieren eines Signals, bereitgestellt durch den detektierbaren Label einer beliebigen einsträngigen DNS, welche mit den Sonden hybridisiert hat, wobei:
- positive Signale gleicher Intensität in beiden Positionen des Mikroarrays, welche den Sonden aus SEQ ID Nr. 1 und SEQ ID Nr. 2 entsprechen, einen Anhalt für das Vorliegen von Streptococcus pneumoniae in der Probe geben;
- ein positives Signal in der Position, welche der Sonde vom Typ SEQ ID Nr. 1 entspricht, und ein Signal in der Position, welche der Sonde aus SEQ ID Nr. 2 entspricht, welches eine Intensität von weniger als 20 % derjenigen Intensität besitzt, welche der Sonde aus SEQ ID Nr. 1 entspricht, Anhalt über das Vorliegen von Streptococcus mitis in der Probe geben; und
- ein positives Signal in der Position, welche nur einer Sonde entspricht, oder kein Signal, in keiner der Positionen, welche den Sonden aus SEQ ID Nr. 1 und 2 entsprechen, Anhalt über das Vorliegen einer anderen Spezies der Gattung Streptococcus gibt.

2. Verfahren nach Anspruch 1, bei welchem zwei Nukleinsäuresequenzen, welche jeweils SEQ ID Nr. 6 und SEQ ID Nr. 7 beinhalten, als PCR-Amplifikations-Primer verwendet werden.

3. Verfahren nach Anspruch 1 oder 2, bei welchem die Sonden aus SEQ ID Nr. 1 und SEQ ID Nr. 2 in einem individuellen Arraygefäß enthalten sind, oder in einem Satz von Arraygefäßen.

4. Kit zur Detektion von Streptococcus pneumoniae in einer Testprobe, bei welchem der Kit ein Arraygefäß oder einen Satz von Arraygefäßen beinhaltet, wobei jedes ein Mikroarray beinhaltet, in welchem beide Sonden aus SEQ ID Nr. 1 und SEQ ID Nr. 2 bereitgestellt sind, und
ein Amplifikationsgemisch, beinhaltend PCR-Amplifikations-Primer und einen detektierbaren Label zum Herstellen gelabelter Amplifikationsprodukte, welche die Region enthalten, welche mit Sonden aus SEQ ID Nr. 1 und SEQ ID Nr 2 hybridisiert.

5. Kit nach Anspruch 4, bei welchem der Kit zudem Reagenzien zum Gebrauch zur Visualisierung der Hybridisierung von Nukleinsäuren an den Sonden des Mikroarrays beinhaltet.

6. Verfahren nach Anspruch 4 oder Anspruch 5, bei welchem das Amplifikationsgemisch zwei Nukleinsäuresequenzen beinhaltet, welche jeweils SEQ ID Nr. 6 und SEQ ID Nr. 7 als PCR-Amplifikations-Primer beinhalten.

7. Satz von Sonden, welcher die Sonden aus SEQ ID Nr. 1 und SEQ ID Nr. 2 beinhaltet.

8. Mikroarray, welches den Satz von Sonden nach Anspruch 7 beinhaltet.

9. Gebrauch des Kits nach den Ansprüchen 4 bis 6, des Satzes von Sonden nach Anspruch 7 oder des Microarrays nach Anspruch 8 zur in vitro-Detektion von Streptococcus pneumoniae.

10. Gebrauch des Kits nach den Ansprüchen 4 bis 6, des Satzes von Sonden nach Anspruch 7 oder des Microarrays nach Anspruch 8 zur Diagnose einer Infektion mit Streptococcus pneumoniae in einer Probe eines Patienten.

11. Gebrauch nach Anspruch 10, bei welchem die Infektion eine septische oder respiratorische Infektion ist.

## Revendications

1. Procédé destiné à la détection de *Streptococcus pneumoniae* dans un échantillon de test, le procédé comprenant :
- la soumission de l'échantillon à une amplification PCR donnant des produits d'amplification qui comportent une région qui s'hybride avec des sondes de SEQ ID N° : 1 et SEQ ID N° : 2, dans lequel un marqueur détectable est introduit dans les produits d'amplification ;
- la transformation de tous produits d'amplification PCR en ADN simple brin ;
- la mise en contact de tout ADN simple brin avec à la fois des sondes de SEQ ID N° : 1 et SEQ ID N° : 2, lesdites sondes étant prévues dans une micromatrice ; et
- la détection d'un signal fourni par le marqueur détectable de tout ADN simple brin qui s'est hybridé avec les sondes, dans lequel :
- des signaux positifs d'intensité égale dans les deux positions de la micromatrice correspondant aux sondes de SEQ ID N° : 1 et SEQ ID N° : 2 indiquent la présence de *Streptococcus pneumoniae* dans l'échantillon ;
- un signal positif dans la position correspondant à la sonde de SEQ ID N° : 1, et un signal dans la position correspondant à la sonde de SEQ ID N° : 2 ayant une intensité inférieure à 20 % de celle correspondant à la sonde de SEQ ID N° : 1, indiquent la présence de *Streptococcus mitis* dans l'échantillon ; et
- un signal positif dans la position correspondant à une seule sonde, ou une absence de signal dans l'une ou l'autre des positions correspondant aux sondes de SEQ ID N° : 1 et 2, indiquent la présence d'une autre espèce du genre *Streptococcus.*

2. Procédé selon la revendication 1, dans lequel deux séquences d'acide nucléique comprenant respectivement SEQ ID N° : 6 et SEQ ID N° : 7 sont utilisées en tant qu'amorces d'amplification PCR.

3. Procédé selon la revendication 1 ou la revendication 2, dans lequel les sondes de SEQ ID N° : 1 et SEQ ID N° : 2 sont incluses dans un récipient de matrices individuel, ou dans un ensemble de récipients de matrices.

4. Trousse pour la détection de *Streptococcus pneumoniae* dans un échantillon de test, dans laquelle ladite trousse comprend un récipient de matrices ou un ensemble de récipients de matrices, chacun comprenant une micromatrice dans laquelle les deux sondes de SEQ ID N° : 1 et SEQ ID N° : 2 sont mises à disposition, et
un mélange d'amplification, comprenant des amorces d'amplification PCR et un marqueur détectable, pour produire des produits d'amplification marqués qui comportent la région qui s'hybride avec des sondes de SEQ ID N° : 1 et SEQ ID N° : 2.

5. Trousse selon la revendication 4, dans laquelle ladite trousse comprend en outre des réactifs pour une utilisation dans la visualisation de l'hybridation d'acides nucléiques avec les sondes de la micromatrice.

6. Trousse selon la revendication 4 ou la revendication 5, dans laquelle le mélange d'amplification comprend deux séquences d'acide nucléique, comprenant respectivement SEQ ID N° : 6 et SEQ ID N° : 7, en tant qu'amorces d'amplification PCR.

7. Ensemble de sondes comprenant des sondes de SEQ ID N° : 1 et SEQ ID N° : 2.

8. Micromatrice comprenant l'ensemble de sondes tel qu'il est défini dans la revendication 7.

9. Utilisation de la trousse selon les revendications 4 à 6, de l'ensemble de sondes selon la revendication 7 ou de la micromatrice selon la revendication 8 pour la détection *in vitro* de *Streptococcus pneumoniae.*

10. Utilisation de la trousse selon les revendications 4 à 6, de l'ensemble de sondes selon la revendication 7 ou de la micromatrice selon la revendication 8 pour le diagnostic d'une infection à *Streptococcus pneumoniae* dans un échantillon provenant d'un patient.

11. Utilisation selon la revendication 10, dans laquelle l'infection est une septicémie ou une infection respiratoire.
